# EUROPEAN PATENT SPECIFICATION

(11) **EP 2 142 561 B1**
(45) Date of publication and mention of the grant of the patent: **26.06.2013**
(21) Application number: 08754185.0
(22) Date of filing: 01.05.2008
(51) Int. Cl.: C07J 1/00, A61K 31/565

(54) **PROCESS FOR PREPARING AROMATASE INHIBITORS**
VERFAHREN ZUR HERSTELLUNG VON AROMATASE-HEMMERN
PROCÉDÉ SERVANT À PRÉPARER DES INHIBITEURS DE L'AROMATASE

(30) Priority: 04.05.2007 US 927626 P
(43) Date of publication of application: 13.01.2010
(73) Proprietor: ScinoPharm Taiwan Ltd., Tainan County 741 (TW)
(72) Inventor: CHEN, WeiYu, Tainan, 710 (TW); CHEN, Shu-Ping, Kaohsiung, 807 (TW)
(74) Representative: Epping - Hermann - Fischer
(86) International application number: PCT/US2008/005646
(87) International publication number: WO 2008/137048

(56) References cited:
- WO-A1-01/04342
- WO-A1-2005/070951
- DD-A1- 264 220
- US-A- 4 808 616
- US-A- 4 824 830
- US-A- 4 876 045
- US-A- 4 990 635
- WOJCIECHOWSKA WANDA ET AL: "Dehydration of exo-hydroxymethyl group in androstane derivative; Optimization and scale-up" 20040101, vol. 48, no. 3-4, 1 January 2004 (2004-01-01), pages 63-74, XP008108245
- GOERLITZER KLAUS ET AL: "Exemestane - synthesis and analysis" DEUTSCHE APOTHEKER ZEITUNG, DEUTSCHER APOTHEKER VERLAG, STUTTGART, DE, vol. 145, no. 37, 15 September 2005 (2005-09-15), pages 69-76, XP002525704 ISSN: 0011-9857
- GOERLITZER K ET AL: "Derivatives of exemestane-synthesis and evaluation of aromatase inhibition" DIE PHARMAZIE, GOVI VERLAG PHARMAZEUTISCHER VERLAG GMBH, ESCHBORN, DE, vol. 61, no. 7, 1 January 2006 (2006-01-01), pages 575-581, XP008108266 ISSN: 0031-7144
- DATABASE CA [Online] CHEMICAL ABSTRACTS SERVICE, COLUMBUS, OHIO, US; 2005, HUANG, HAIYAN ET AL: "Process for synthesis of 6-methyleneandrosta-1,4-diene-3,17-dione" XP002572872 retrieved from STN Database accession no. 2005:626970 & CN 1 415 624 A (NANJING CHANG'AO PHARMACEUTICAL SCIENCE AND TECHNOLOGY CO., LTD., PEOP) 7 May 2003 (2003-05-07)

## Description

### RELATED APPLICATIONS

This application claims priority from U.S. Provisional Patent Application Serial Number 60/927,626 which was filed on May 4, 2007. The entire content of Provisional Patent Application Serial Number 60/927,626 is incorporated herein by reference.

### BACKGROUND OF THE INVENTION

### 1. Field of the Invention

The invention relates to methods of making aromatase inhibitors 6-alkylidenandrosta-1,4-diene-3,17-dione derivatives, such as exemestane, and new polymorphs of exemestane.

### 2. Description of the Related Art

Estrogens are the hormones involved in the pathogenic cellular changes associated with the growth of some hormone-dependent cancers, such as breast, endometrial and ovarian carcinomas. Estrogens are also involved in the pathogenesis of benign prostatic hyperplasia. Endogenous estrogens are ultimately formed from either androstenedione or testosterone as immediate precursors. The reaction of central importance is the aromatization of the steroidic ring A, which is performed by the enzyme aromatase. As aromatization is a unique reaction and the last in the series of steps in the biosynthesis of estrogens, it has been envisaged that an effective inhibition of the aromatase, resulting from compounds capable of interacting with the aromatizing steps, may have useful application for controlling the amount of circulating estrogens, estrogen-dependent processes in reproduction, and estrogen-dependent tumors. See U.S. Patent No. 4,904,650, col. 1, lines 10-30.

6-alkylidenandrosta-1,4-diene-3,17-dione derivatives, such as exemestane, are reported to be endowed with an aromatase-inhibiting actions. Exemestane (brand name Aromasin®) is chemically described as 6-methylenandrosta-1, 4-diene-3,17-dione. Its molecular formula is C₂₀H₂₄O₂ and its structural formula is as follows :

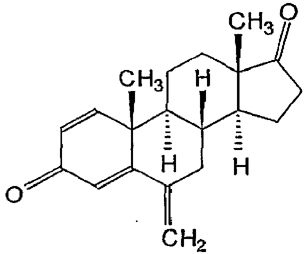

U. S. Patent No. 4,876, 045 teaches a method of preparing 6-methylene derivatives of androsta-1, 4-diene-3,17-diones by reacting a 17-hydroxy precursor with formaldehyde and an amine, and then oxidizing the resulting compound. U. S. Patent No. 4,990, 635 teaches a process for making 6-methylene derivatives of androsta-1,4-diene-3, 17-diones by reacting androsta-3,5-diene-17-one with formaldehyde and an amine, and then dehydrogenating the resulting compound. Published PCT Patent Application WO 2005/070951 discloses a two-step process of making exemestane. The process comprises: 1) reacting a 6-hydroxymethyl derivative with the following formula: 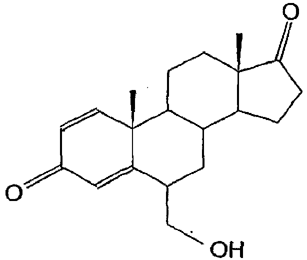 with a deprotonating agent (e.g., a trialkylamine) and a R₅SO₂X wherein R₅ is C₁-C₅ alkyl and X is halogen in a solvent such as dichloromethane to obtain a compound of mesylate intermediate with the following formula: 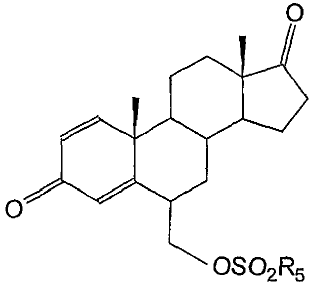 and 2) then reacting the mesylate intermediate with a base in a solvent to produce exemestane.

Wanda et al.: "Dehydration of exo-hydroxymethyl group in androstane derivative; Optimization and scale-up", (Polish Journal of Applied Chemistry, XLVII, No. 3-4, pages 63 to 74 (2004)) describe the dehydration reaction of 6β-hydroxymethyl-androsta-1,4-diene-3,17-dione to yield the olefin 6-methyleneandrosta-1,4-diene-3,17-dione.

Although various methods for preparing aromatase inhibitors, such as exemestane, have been described in the art, there is a continuing need for a simple and efficient method for preparing aromatase inhibitors, such as exemestane, in commercial quantities with high yield and high purity.

### SUMMARY OF THE INVENTION

Accordingly, Applicants provide a process of making an aromatase inhibitor of formula (I) 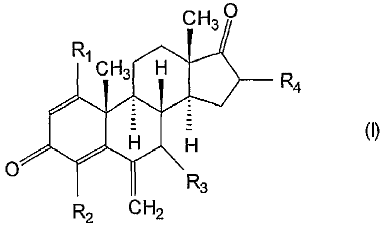 wherein each of R¹, R², R³, R⁴, independently, is hydrogen, halogen, or C₁-C₆ alkyl. In one form, the aromatase inhibitor is exemestane, wherein each of R₁, R₂, R₃, R₄ is hydrogen.

In accordance with one embodiment of the present invention, a compound of formula (II) 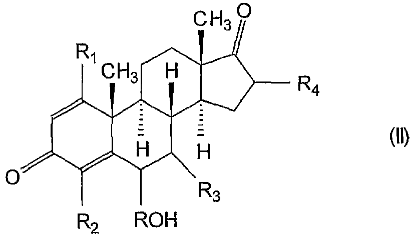 wherein each of R¹, R², R³, R⁴ , independently, is hydrogen, halogen, or C₁-C₆ alkyl, and R is methylene, is reacted with an acid, such as para-toluenesulfonic acid, sulfuric acid, camphorsulfonic acid, hydrochloric acid, acetic acid or trifluoracetic acid, in the presence of a organic solvent, selected from the group consisting of: toluene, benzene, xylenes, ethyl acetate, methyl isobutyl ketone (MIBK), or a mixture thereof, to produce the aromatase inhibitor of formula (I).

The synthesis of the compound of formula (I) is carried out at a temperature of 60°C to 90°C.

Compared to the prior art process, the process in accordance with the present invention produces a higher yield of formula (I). Specifically, according to the process disclosed in U.S. Patent No. 4,876,045, the yield of preparing 6-methylene derivatives of androsta-1, 4-diene-3,17-diones is 30.7% (example 1), and the yield of preparing exemestane from the 6-methylene derivatives is 79% (example 2). However, according to the historical data collected from Applicants' production line, the yield of preparing 6-hydroxymethyl-androsta-1,4-diene-3,17-dione is about 80%, and the yield of preparing exemestane from 6-hydroxymethyl-androsta-1,4-diene-3,17-dione is about 80 to 90% (see Examples below).

In addition, the two-step process disclosed in published PCT Patent Application No. WO2005/070951 needs to use several kinds of reagents and solvents, which may increase the cost and cause more impurities carried from those reagents and solvents. On the contrary, in addition to the reactant, the one-step process in accordance with the present invention only requires the catalyst amount of acid in the presence of a suitable solvent. The one-step process can be performed by a simple operation. Therefore, the advantages of the present invention include simple operation, low cost, high purity, and high yield.

The present application also provides a new crystalline form of exemestane, which is not claimed. The powder X-ray diffraction pattern and the Infrared spectrum of this crystalline form of exemestane are herein disclosed.

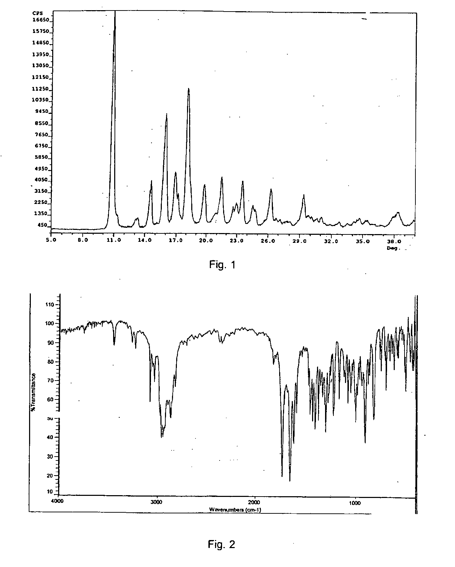

The crystalline exemestane is characterized by a powder X-ray powder diffraction pattern having peaks at 10.9±0.1, 16.0±0.1, 18.2±0.1 2-theta degree. Preferably, the crystalline exemestane exhibits further X-ray powder diffraction pattern peaks at 19.6±0.1, 19.8±0.1, 21.5±0.1, 23.5±0.1, 26.3±0.1, and 29.3±0.1 2-theta degree. More preferably, the crystalline solid exemestane exhibit a powder X-ray diffraction pattern as depicted in Fig. 1.

Preferably, the crystalline solid exemestane exhibits an infrared spectrum with bands at 1732 ± 2 cm⁻¹, 1658 ±2 cm⁻¹, 1620 ±2cm⁻¹. More preferably, the crystalline solid exemestane exhibit an infrared spectrum as depicted in Fig. 2.

The stability of various crystalline solid exemestane samples obtained by a process in accordance with the present invention has been tested under various conditions. HPLC was used to determine the degree of degradation of exemestane over time. The samples of crystalline solid exemestane were respectively held at 25°C/60%RH and 40°C/75%RH for six months. We tested the purities of these samples by HPLC and observed the changes of their purities. The total impurities of these samples collected after 6 months stability test and before the stability test (Day 0) were all less than 0.05%.

The exemestane produced by a process in accordance with one embodiment of the present invention has a purity of at least 95% as determined based on peak area percentage obtained by HPLC analysis. In fact, exemestane produced by the one-step process in accordance with the present invention can achieve high purity without further re-crystallizing. Referring to Examples 1-3 below, the crude exemestane (before re-crystallizing) can achieve the purity more than 95% HPLC Peak Area.

The various features of novelty which characterize the invention are pointed out with particularity in the claims annexed to and forming a part of the disclosure. For a better understanding of the invention, its operating advantages, and specific objects attained by its use, reference should be had to the drawings and descriptive matter in which there are illustrated and described preferred embodiments of the invention.

### BRIEF DESCRIPTION OF THE DRAWINGS

### In the drawings:

Fig. 1 is a representative powder x-ray diffraction pattern of crystalline solid exemestane produced by a process in accordance with one embodiment of the present invention.

Fig. 2 is a representative IR spectrum of crystalline solid exemestane produced by a process in accordance with one embodiment of the present invention.

### DETAILED DESCRIPTION OF THE PRESENTLY PREFERRED EMBODIMENTS

In accordance with one embodiment of the present invention, exemestane can be prepared from a 6-hydroxymethyl intermediate in a one step process as shown in the following scheme:

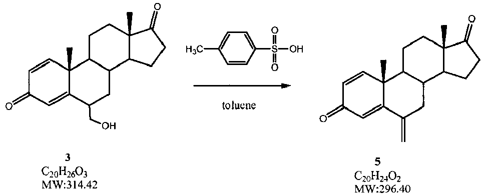

The general conditions of the above synthetic process are 80-90 ° C, preferably about 330 torr, preferably about 10 vol. parts toluene, preferably and 5-15 wt.% para-toluenesulfonic acid (p-TsOH) as the reagent and compound 3 as the reactant.

In comparison, in the process of WO 2005/070951, to make exemestane from a 6-hydroxymethyl intermediate, there must be two steps as shown in the following reaction scheme:

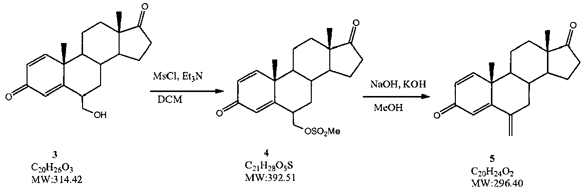

The intermediates involved in the present invention may be prepared by any suitable method, e.g., a method described in the literature. For example, U. S. Patent No. 3,274,176, discloses a process for making 1,3-dipyrrolidyl-Δ^{3,5}-androstadiene-17-one (compound 2) in which-Δ^{1,4}-androstadiene-3, 17-dione (compound 1) is refluxed with pyrrolidine and the residue is crystallized in methanol to obtain 1,3-dipyrrolidyl-Δ^{3,5}-androstadiene-17-one (compound 2). In German patent DD 258820, 6-hydroxymethyl-androsta-1, 4-diene-3,17-dione (compound 3) is prepared from androsta-1, 4-diene-3,17-dione (compound 1) via 1, 3-dipyrrolidinoandrosta-3,5- dien-17-one (compound 2). See the following scheme.

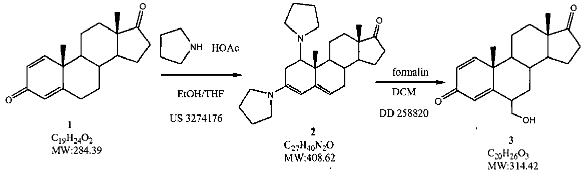

The following examples are presented to further illustrate the present invention and not intended to limit the invention in any way. Examples 1-4 illustrate the synthesis of exemestane produced by a process in accordance with some embodiments of the present invention. Comparative Examples 1-2 are provided to illustrate the two-step synthetic process similar to the process disclosed in WO 2005/070951. Examples 5-16 illustrate the recrystallization of crude exemestane produced by a process in accordance with some embodiments of the present invention.

### Example 1

6-hydroxymethyl-androsta-1,4-diene-3,17-dione (10.00g), p-toluenesulfonic acid (0.50g) and toluene(60 mL) was added to a suitable reactor. The mixture was heated under reduced pressure at about 315 torr to boiling (the boiling point of toluene is about 80-90°C at 315 torr) to remove water by dean-stark for not less than 4 hours. After the reaction was complete, the mixture was cooled down to 60°C or below. Then 2.5% sodium bicarbonate (50 mL) was added to wash the mixture. The reaction mixture was filtered through precoat celite bed before phase separated. The aqueous phase was back-extracted by toluene (20 mL).

The two organic phases were combined together. Water (25 mL) was then added to the organic phase to wash. After the wash, toluene was removed under reduced pressure and at a temperature not more than 80°C until the volume was reduced to about 25 mL. N-heptane was added to the reaction mixture (25 mL) to produce a solid. It was held at the cloudy point for not less than (NLT) 0.5 hr. Then, it was cooled to room temperature and held for more than 2 hrs. The slurry was filtered and washed with toluene/n-heptane(10 mL/15mL) to give 8.75 g wet cake. The cake was dried under vacuum at below 70 °C to give 7.85 g crude exemestane. The purity of the crude exemestane was about 98% as determined based on peak area percentage obtained by HPLC analysis. All reported purity of exemestane in the present disclosure is based on HPLC unless indicated otherwise.

The alternative way to isolate exemestane from the slurry is described as follows. The slurry is filtered without washed and dried under vacuum to provide the wet cake. The wet cake and acetonitrile are charged into a suitable reactor with heating to dissolve. The resulting mixture is cooled to about 5°C and then filtered to provide purified exemestane.

### Example 2

To a suitable reactor was added 2.00 g of 6-hydroxymethyl-androsta-1,4-diene-3,17-dione, 0.10 g camphorsulfonic acid, and 20 mL toluene to mix well. The mixture was heated up to about 90°C and held at that temperature for about 7 hours. After the reaction was complete, 10 mL 2.5 % sodium bicarbonate solution was added to wash. The aqua phase was back-extracted with 5 mL toluene. The two organic phases were combined together and distilled until dry. 20 mL isopropanol was added to the resulting solid and heated to reflux to dissolve. The solution was cooled down to 0°C. After cooling, the slurry was filtered and washed with 4 mL cold isopropanol. The solid was dried under vacuum to give 1.21 g exemestane with a purity of 97% as determined based on peak area percentage obtained by HPLC analysis.

### Example 3

To a suitable reactor was added 2.00 g 6-hydroxymethyl-androsta-1,4-diene-3,17-dione, 0.10 g p-toluenesulfonic acid monohydrate, and 20 mL toluene to mix well. The mixture was heated up to about 90°C and held at that temperature for about 3 hours. After the reaction was complete, 20 mL 2.5 % sodium bicarbonate solution was added to wash. The organic phase was distilled to almost dry. 20 mL methanol and 2 mL 5% sodium bicarbonate solution was added to the resulting solid. The slurry was distilled to about 22 mL residue. 25 mL water was added to the solution. The solution was cooled down to ambient temperature. After cooling, the slurry was filtered and washed with 4 mL 50 % (v/v) methanol aqua solution. The solid was dried under vacuum to give 1.27 g exemestane with a purity of 96% as determined based on peak area percentage obtained by HPLC analysis.

### Example 4

6-hydroxymethyl-androsta-1,4-diene-3,17-dione (2.0 g), (D)(+)-10-camphorsulfonic acid (0.1g) and ethyl acetate (30 ml) were added into a suitable reactor. The resulting mixture was reacted under reflux (about 70 to 80°C) for 32 hours. After reaction was complete, the resulting mixture was dried under vacuum, and toluene (30 ml) was added. The resulting solution was extracted by sodium bicarbonate solution (10 ml) and water (5 ml). The organic layer was dried under vacuum, and then methanol (20 ml) was added. Water (20 ml) was added to the resulting mixture, and then the mixture was cooled down to room temperature. The slurry was filtered, and then the wet cake was dried to give exemestane.

### Comparative Example 1

5.00 g 6-hydroxymethyl-androsta-1,4-diene-3,17-dione and 80 mL pyridine were added to a suitable reactor to mix well. The mixture was cooled to 0°C. 6.20 g toluenesulfonic chloride was added and held at 0°C for 2 days. After the reaction was complete, 135 mL water was added to quench the reaction. 20 mL methylene chloride was added to extraction. The aqua phase was back-extracted by 20 mL methylene chloride. The two organic phases were combined and washed with 40 mL Brine/water (v/v=1/1). The organic phase was distilled until 30 mL residue was remained. 60 mL water/methanol(v/v=1/1) and 1.32 g potassium hydroxide were added to the mixture and heated to 65°C. The mixture was held at 65°C for about 2 hours. After the reaction was completed, 130 mL water was added and cooled to ambient temperature. The slurry was filtered and washed with 30 mL methanol/water (v/v=1/1). The cake was dried under vacuum below 50°C to give 2.35 g exemestane with a purity of about 87 % as determined based on peak area percentage obtained by HPLC analysis.

### Comparative Example 2

5.00 g 6-hydroxymethyl-androsta-1,4-diene-3,17-dione and 80 mL pyridine were added to a suitable reactor. The mixture was cooled to 0°C. 6.20 g p-toluenesulconic chloride was added and held at 0°C for 2 days. After the reaction was complete, 135 mL water and 20 mL methylene chloride were added to extraction. The aqua phase was back-extracted by 20 mL methylene chloride. The two organic phases were combined and washed with 40 mL brine/water(v/v=1/1) (brine is a saturated sodium chloride aqueous solution). The organic phase was distilled until 30 mL residue remained. 30 mL water and 30 mL methanol and 1.32 g potassium hydroxide were added to the mixture and heated to 65 °C. The mixture was held at 65°C for about 2 hours. After the reaction was completed, 80 mL water was added and cooled to ambient temperature. The slurry was filtered and washed with 20 mL methanol/water (v/v=1/1). The cake was dried under vacuum below 50°C to give 1.16 g exemestane with a purity of 85 % as determined based on peak area percentage obtained by HPLC analysis.

### Example 5

To a suit reactor was charged crude exemestane (3.0 g) and acetone (15 mL). The resulting mixture was stirred and warmed up to 50-60°C until the solid was almost dissolved. Water (9 mL) was charged at 50-60°C and stirred at that temperature for 0.5 hour. The resulting slurry was cooled to 20-30°C at a rate of 15°C /hr and kept at 20-30°C for NLT 1 hour. The slurry is filtered, and then the wet cake was dried at 50°C to get about 2.72 g of pure exemestane in an expected yield of 85-95%, based on weight.

### Example 6

To a suitable reactor was charged crude exemestane (3.0 g) toluene (9 mL). The resulting mixture was stirred and warmed up to 90-100°C until the solid was almost dissolved. Heptane (9 mL) was charged at 90-100°C and stirred at that temperature for 0.5 hour. The resulting slurry was cooled to 20-30°C at a rate of 15°C /hr and kept at 20-30°C for at least 1 hour. The slurry was filtered, then the wet cake was dried at 50°C to get about 2.81 g of pure exemestane in an expected yield of 85-95%

### Example 7

To a suitable reactor was charged crude exemestane (3.0 g) and ACN (acetonitrile) (12 mL). The resulting mixture was stirred and warmed up to 70-80°C until the solid was almost dissolved. Water (15 mL) was charged at 70-80°C and stirred at that temperature for 0.5 hour. The resulting slurry was cooled to 20-30°C at a rate of 15°C /hr and kept at 20-30°C for NLT 1 hour. The slurry was filtered, then the wet cake was dried at 50°C to get 2.79 g of pure exemestane in an expected yield of 85-95%.

### Example 8

To a suitable reactor was charged crude exemestane (20 g) and CH₂Cl₂ (106 g). The resulting mixture was stirred and warmed up to 40-50°C until the solid was almost dissolved. Heptane (41.0 g) was charged at 40-50°C and stirred at that temperature for 0.5 hour. The resulting slurry was cooled to 20-30°C at a rate of 15°C /hr and kept at 20-30°C for NLT 1 hour. The slurry was filtered, and then the wet cake was dried at 50°C to get about 16.5 g of pure exemestane in an expected yield of 80-90%.

### Example 9

To a suitable reactor was charged crude exemestane (5.0 g), and CH₂Cl₂ (20 mL). The resulting mixture was stirred and warmed up to 40-50°C until the solid was substantially dissolved. MTBE (13 mL) was charged at 40-50°C and stirred at that temperature for 0.5 hour. The resulting slurry was cooled to 20-30°C at a rate of 15°C /hr and kept at 20-30°C for NLT 1 hour. The slurry was filtered, and then the wet cake was dried at 50°C to get about 4.7 g of pure exemestane in an expected yield of 85-95%.

### Example 10

To a suitable reactor was charged crude exemestane (3.0 g) and ethyl acetate (EtOAc) (15 mL). The resulting mixture was stirred and warmed up to 65-75°C until the solid was almost dissolved. Heptane (12 mL) was charged at 65-75°C and stirred at that temperature for 0.5 hour. The resulting slurry was cooled to 20-30°C at a rate of 15°C /hr and kept at 20-30°C for NLT 1 hour. The slurry was filtered, and then the wet cake was dried at 50°C to get about 2.25 g of pure exemestane in an expected yield of 70-80%.

### Example 11

To a suitable reactor was charged crude exemestane (3.0 g) and 95% ethyl alcohol EtOH (12 mL). The resulting mixture was stirred and warmed up to 75-85°C until the solid was substantially dissolved. Water (9 mL) was charged at 75-85°C and stirred at that temperature for 0.5 hour. The resulting slurry was cooled to 20-30°C at a rate of 15°C /hr and kept at 20-30°C for at least 1 hour. The slurry was filtered, and then the wet cake was dried at 50°C to get about 2.82 g of pure exemestane in an expected yield of 85-95%.

### Example 12

To a suitable reactor was charged crude exemestane (2.0 g) and acetic acid (6 mL). The resulting mixture was stirred and warmed up to 40-50°C until the solid was almost dissolved. Water (6 mL) was charged at 40-50°C and stirred at that temperature for 0.5 hour. The resulting slurry was cooled to 20-30°C at a rate of 15°C /hr and kept at 20-30°C for at least 1 hour. The slurry was filtered, and then the wet cake was dried at 50°C to get about 1.73 g of pure exemestane in an expected yield of 80-90%.

### Example 13

To a suitable reactor was charged crude exemestane (2.0 g), IPA (isopropyl alcohol) (10 mL). The resulting mixture was stirred and warmed up to 75-85°C until the solid is almost dissolved. The resulting slurry was cooled to 20-30°C at a rate of 15°C/hr and kept at 20-30°C for at least 1 hour. The slurry was filtered, and then the wet cake was dried at 50°C to get about 1.82 g of pure exemestane in an expected yield of 85-95%.

### Example 14

To a suitable reactor was charged crude exemestane (3.0 g), MeOH (18 mL). The resulting mixture was stirred and warmed up to 55-65°C until the solid was almost dissolved. Water (6 mL) was charged at 55-65°C and stirred at that temperature for 0.5 hour. The resulting slurry was cooled to 20-30°C at a rate of 15°C /hr and kept at 20-30°C for NLT 1 hour. The slurry was filtered, and then the wet cake was dried at 50°C to get about 2.68 g of pure exemestane in an expected yield of 85-95%.

### Example 15

To a suitable reactor was charged crude exemestane (2.0 g) and n-butanol (6 mL). The resulting mixture was stirred and warmed up to 90-100°C until the solid was almost dissolved. The resulting slurry was cooled to 20-30°C at a rate of 15°C /hr and kept at 20-30°C for at least 1 hour. The slurry was filtered, and then the wet cake was dried at 50°C to get about 1.67 g of pure exemestane in an expected yield of 80-90%.

### Example 16

To a suitable reactor was charged crude exemestane (3.0 g) and THF (tetrahydrofuran) (9 mL). The resulting mixture was stirred and warmed up to 40-50°C until the solid was almost dissolved. Water (6 mL) was charged at 40-70°C and stirred at that temperature for 0.5 hour. The resulting slurry was cooled to 20-30°C at a rate of 15°C /hr and kept at 20-30°C for at least 1 hour. The slurry was filtered, and then the wet cake was dried at 50°C to get about 2.54 g of pure exemestane in an expected yield of 80-90%.

As shown above, the purity of the exemestane obtained in the two-step process of comparative Examples 1-2 is much lower than that obtained in accordance with the present invention (e.g. Examples 1-4). Therefore, the side products or impurities formed in the one-step process disclosed in the present invention would be much lower than the two-step process. Especially, Example 1 recites a preferable process to prepare exemestane. The higher yield can be obtained when applying toluene as the reaction solvent and n-heptane as the anti-solvent for precipitating. And using acetonitrile to isolate exemestane from the resulting mixture can achieve higher purity and help to decolor.

The crude exemestane produced by the processes recited in examples 1-4 can be re-crystallized by the processes recited in examples 5-16 to give the crystalline form exhibiting the XRD pattern and IR spectrum shown in Figs. 1-2.

The procedure of XRD test used for obtaining Fig.1 is as follows. The test sample was milled and homogenously put on the tray of the X-ray machine, Scintag X2 Advance Diffraction, tested at continuous scan rate of 2.00 Deg/min, with range 5.00-40.00 (Deg.) and at a wavelength of 1.540562.

The procedure of IR test used for obtaining Fig. 2 is as follows. We weighed about 3 mg of sample and disperse the sample homogenously in 300 mg dry KBr, and then, immediately recorded the spectrum between 400 to 4000 cm-1 by diffuse reflectance. We performed a single test on the sample. The IR machine was Nicolet, Magna-IR 560 Spectrometer. The number of sample scans was 32. The number of background scans was 32. The resolution was 4. The sample gain was 8. The mirror velocity was 0.6329. The aperture was 100.

## Claims

1. A process of making an aromatase inhibitor of formula (I) 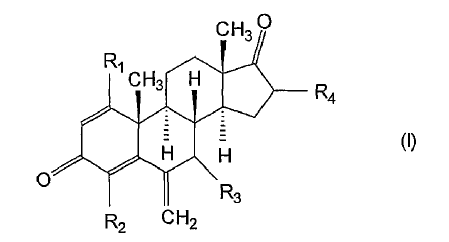 wherein each of R₁, R₂, R₃, R₄, independently, is hydrogen, halogen, or C₁-C₆ alkyl, comprising: reacting a compound of formula (II) 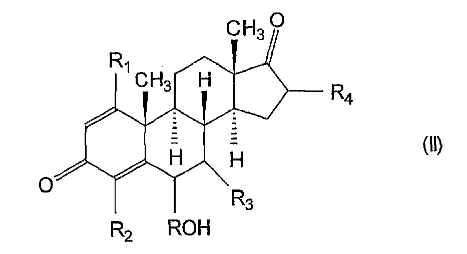 wherein R₁, R₂, R₃, R₄ are as defined above and R is methylene, with an acid in the presence of a solvent at a reaction temperature of 60 to 90°C to produce the aromatase inhibitor of formula (I), wherein the solvent is an organic solvent selected from the group consisting of toluene, benzene, xylenes, ethyl acetate, methyl isobutyl ketone, and mixtures thereof.

2. The process of claim 1 wherein the acid is selected from the group consisting of para-toluenesulfonic acid, sulfuric acid, camphorsulfonic acid, hydrochloric acid, acetic acid, trifluoracetic acid, and mixtures thereof.

3. The process of any of the claims 1 to 2 wherein each of R₁, R₂, R₃, and R₄ is hydrogen.

4. The process of claim 1 wherein the acid is para-toluenesulfonic acid.

5. The process of claim 1 wherein the solvent is toluene.

6. The process of any of the claims 1 to 5 further comprising a step of adding an antisolvent to the mixture formed after the reaction of the compound of formula (II) and the acid to precipitate the aromatase inhibitor of formula (I).

7. The process of claim 6 wherein the anti-solvent is n-heptane.

8. The process of any of the claims 6 or 7 further comprising the steps of :
(a) collecting the precipitates of aromatase inhibitor of formula (I);
(b) dissolving the collected precipitates with acetonitrile under an elevated temperature; and
(c) cooling the resulting mixture of step (b) to precipitate the aromatase inhibitor of formula (I).

9. The process of any of the claims 1 to 8 wherein the reacting is carried out at a temperature of 80 to 90°C.

## Patentansprüche

1. Verfahren zur Herstellung eines Aromataseinhibitors der Formel (I) 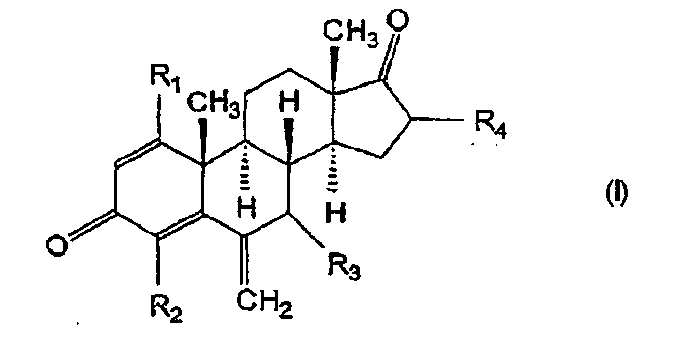 worin R₁, R₂, R₃ und R₄ jeweils unabhängig voneinander für Wasserstoff, Halogen oder C₁-C₆-Alkyl stehen, umfassend die Reaktion einer Verbindung der Formel (II) 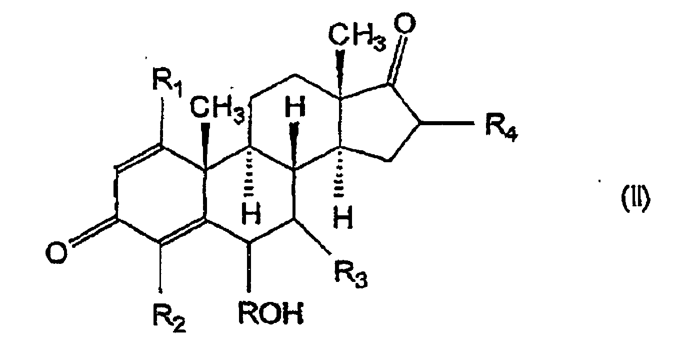 worin R₁, R₂, R₃ und R₄ wie oben definiert sind und R für Methylen steht, in Gegenwart eines Lösungsmittels bei einer Reaktionstemperatur von 60 bis 90°C mit einer Säure zu dem Aromataseinhibitor der Formel (I), wobei es sich bei dem Lösungsmittel um ein organisches Lösungsmittel aus der Gruppe bestehend aus Toluol, Benzol, Xylolen, Essigsäureethylester, Methylisobutylketon und Mischungen davon handelt.

2. Verfahren nach Anspruch 1, bei dem man die Säure aus der Gruppe bestehend aus para-Toluolsulfonsäure, Schwefelsäure, Camphersulfonsäure, Salzsäure, Essigsäure, Trifluoressigsäure und Mischungen davon auswählt.

3. Verfahren nach einem der Ansprüche 1 bis 2, bei dem R₁, R₂, R₃ und R₄ jeweils für Wasserstoff stehen.

4. Verfahren nach Anspruch 1, bei dem es sich bei der Säure um para-Toluolsulfonsäure handelt.

5. Verfahren nach Anspruch 1, bei dem es sich bei dem Lösungsmittel um Toluol handelt.

6. Verfahren nach einem der Ansprüche 1 bis 5, umfassend ferner den Schritt des Zugebens eines Antilösungsmittels zur Ausfällung des Aromataseinhibitors der Formel (I) zu einer Mischung, die nach der Umsetzung der Verbindung der Formel (II) und der Säure gebildet wurde.

7. Verfahren nach Anspruch 6, bei dem es sich bei dem Antilösungsmittel um n-Heptan handelt.

8. Verfahren nach einem der Ansprüche 6 oder 7, umfassend:
(a) das Sammeln der Niederschläge vom Aromataseinhibitor der Formel (I);
(b) das Lösen der gesammelten Niederschläge bei erhöhter Temperatur mit Acetonitril und
(c) das Abkühlen des erhaltenen Gemisches aus Schritt (b) zur Ausfällung des Aromataseinhibitors der Formel (I).

9. Verfahren nach einem der Ansprüche 1 bis 8, bei dem man die Umsetzung bei einer Temperatur von 80 bis 90°C durchführt.

## Revendications

1. Procédé de fabrication d'un inhibiteur d'aromatase de formule (I) 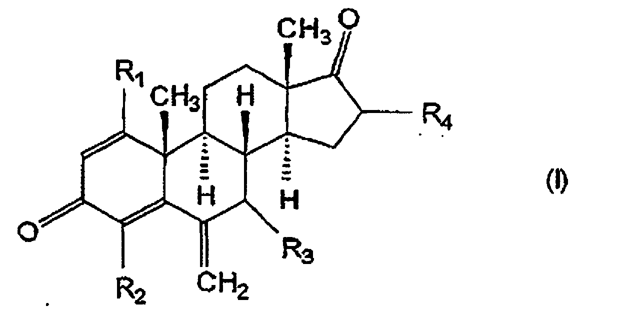 dans laquelle chacun des radicaux R₁, R₂, R₃, R₄, représente indépendamment un atome d'hydrogène, d'halogène, ou un groupe alkyle en C₁-C₆, comprenant les étapes consistant à : faire réagir un composé de formule (II) 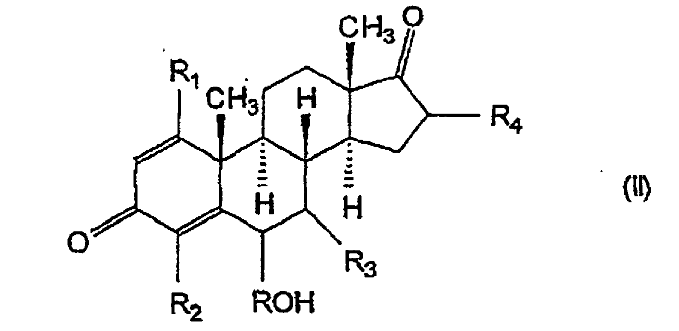 dans laquelle R₁, R₂, R₃, R₄ sont tels que définis ci-dessus et R représente un groupe méthylène, avec un acide en présence d'un solvant à une température réactionnelle de 60 à 90°C pour produire l'inhibiteur d'aromatase de formule (I), dans lequel le solvant est un solvant organique choisi dans le groupe constitué par le toluène, le benzène, les xylènes, l'acétate d'éthyle, la méthylisobutylcétone, et des mélanges de ceux-ci.

2. Procédé selon la revendication 1 dans lequel l'acide est choisi dans le groupe constitué par l'acide para-toluènesulfonique, l'acide sulfurique, l'acide camphosulfonique, l'acide chlorhydrique, l'acide acétique, l'acide trifluoracétique, et des mélanges de ceux-ci.

3. Procédé selon l'une quelconque des revendications 1 à 2 dans lequel chacun des radicaux R₁, R₂, R₃ et R₄ représente un atome d'hydrogène.

4. Procédé selon la revendication 1 dans lequel l'acide est l'acide para-toluènesulfonique.

5. Procédé selon la revendication 1 dans lequel le solvant est le toluène.

6. Procédé selon l'une quelconque des revendications 1 à 5 comprenant en outre une étape d'addition d'un anti-solvant au mélange formé après la réaction du composé de formule (II) et de l'acide pour précipiter l'inhibiteur d'aromatase de formule (I).

7. Procédé selon la revendication 6 dans lequel l'anti-solvant est le n-heptane.

8. Procédé selon l'une quelconque des revendications 6 ou 7 comprenant en outre les étapes consistant à :
(a) recueillir le précipité d'inhibiteur d'aromatase de formule (I) ;
(b) dissoudre le précipité recueilli avec de l'acéto-nitrile à une température élevée ; et
(c) refroidir le mélange résultant de l'étape (b) pour précipiter l'inhibiteur d'aromatase de formule (I).

9. Procédé selon l'une quelconque des revendications 1 à 8 dans lequel la réaction est réalisée à une température de 80 à 90°C.
